Europäisches Patentamt

European Patent Office

Office européen des brevets

(19)

(11) Veröffentlichungsnummer: **0 229 874**
**A1**

## (12) EUROPÄISCHE PATENTANMELDUNG

(21) Anmeldenummer: **86111979.0**

(22) Anmeldetag: **29.08.86**

(51) Int. Cl.⁴: **A61N 1/16**

(30) Priorität: **23.11.85 DE 3541480**

(43) Veröffentlichungstag der Anmeldung:
**29.07.87 Patentblatt 87/31**

(84) Benannte Vertragsstaaten:
**AT CH DE FR GB LI**

(71) Anmelder: **ORDO - Stiftung**
**Nonnenweg 66**
**CH-4055 Basel(CH)**

(72) Erfinder: **Palm, Hubert, Dr. Med.**
**Linzgaublick 2**
**D-7750 Konstanz-Wallh.(DE)**

(54) **Schutzgerät.**

(57) Die Schutzwirkung und Heilwirkung des Gerätes für alles Leben gründet in seiner qualitativen Bio-Baugleichheit mit dem harmonikalen System des Kosmos und speziell -nach Kepler-des Sonnensystemes. Seine Konstruktion geht aus von der Annahme aller Hochreligionen und Hochkulturen, dass der gute Teil des Kosmos im Ganzen -als Schöpfung Gottes-und daher auch mit all seinen Teilen eine integrale Lebenseinheit ist. Lebensqualitativ baugleiche, insbesondere elektromagnetisch schwingungsfähige harmonische Geräte müssen daher bio-logisch die gleichen kosmischen Lebenskräfte verarbeiten wie induktiv vermitteln, die erfahrungsgemäss im Kosmos überall anwesend sind, insbesondere in Schwingungsform.

FIG. 2

EP 0 229 874 A1

## Schutzgerät

Aus der Umwelt dringen auf die lebensqualifizierten realen Einheiten wie die Menschen, Tiere, Pflanzen, Kristalle, insbesondere auf Nahrungsmittel, Getränke, Textilien, Bauten, Genussmittel, Heilmittel, Hygienika wie Kosmetika, auf Geräte etc. vielerlei disharmonische und daher schädliche Wirkungen ein in Form von Feldern, Strahlen und Strömen, insbesondere in materieller Gestalt. Auch in den realen kosmischen Einheiten selbst entstehen disharmonische Wirkungen. Es besteht daher die Grundaufgabe, sich möglichst universell gegen alle äusseren und inneren Disharmonien zu - schützen und die Qualitäten des eigenen Lebens zu verbessern, sowie auch die Qualitäten der Einheiten zu verbessern, die in der Umwelt wirken, speziell auf den Menschen.

Nach dem Stand der Technik existieren viele spezielle Geräte, die in irgend einem Lebensbereich entweder nur zu schützen oder auch die Situation zu verbessern suchen. Modernerweise arbeiten einige Geräte mit Schwingungskreisen, Kondensatoren und Widerständen, welche von einer eigenen Stromquelle oder von den Störschwingungen angeregt werden und diese dann durch Interferenz oder anderes auszugleichen suchen. Sie sind zur Entstörung von Elektrogeräten, Autos etc. weithin gesetzlich vorgeschrieben. Andere Geräte arbeiten mit der Wirkung von Formen wie z.B. mit den Formen der Cheopspyramide gemäss dem tschechischen Patent Nr. 91 304.

Soweit bekannt ist die Wirksamkeit fast all dieser Geräte sehr beschränkt in der Qualität, insofern sie auch selber mehr oder weniger weit stören können durch ihre eigenen Disharmonien. Und sie sind meist eng spezialisiert auf ein bestimmtes Wirkfeld. Und ihre Entstörwirkung ist auch meist quantitativ schwach. Die Formwirkung der Cheopspyramide ist zwar schon sehr allgemein, aber noch nicht genügend universal. Sie ist zu wenig wirksam in der guten Grundstruktur des Sonnensystemes und in der guten dreieinigen Urstruktur des Kosmos, welche alles kosmische Leben begründet.

Es besteht daher die Aufgabe, ein besser wirksames Gerät zu entwickeln, das die universalen Grundharmonien des Kosmos, speziell des Sonnensystemes in seinen Formen enthält und daher harmonischer, universeller und stärker als das Gerät der Cheopspyramide und andere Geräte wirkt. Ein universales Gerät kann ausnahmslos alle realen Einheiten und all deren gute Funktionen harmonierend beeinflussen. Ein solches Gerät muss logischerweise gemäss seiner Natur die Doppelfunktion haben, einerseits die Entwicklung vorhandener harmonischer und somit lebensgerechter Funktionen zu fördern, insbesondere im Sinne der Verbesserung und Stärkung, andererseits vor störenden Einwirkungen aus der Umwelt und auch Innenwelt zu schützen. Die Schutzwirkung entsteht einerseits direkt gegenüber der Störwirkung durch eine harmonierende und somit ausgleichende, entstörende Gegenwirkung wie im Sinne der Interferenzlöschung, andererseits indirekt durch Qualifizierung wie Stärkung und Festigung der harmonischen Funktionen der zu schützenden Einheit.

Solch ein Gerät ist entwickelt worden. Es enthält erfindungsgemäss mehrere fundamentale harmonische Strukturelemente des Kosmos und speziell des Sonnensystemes. Diese sind schon einzeln wirksam. Sie können noch höher wirksam kombiniert werden. Da jede reale Einheit ein qualifiziertes Feld hat, da die Felder einander beeinflussend durchdringen und da jedes Feld kausalgesetzlich sein harmonisches oder/und disharmonisches Wesen agierend und reagierend wirkt, so wirkt alles im Kosmos auf alles. Das lehrt die Physik der Hochkulturen seit jeher.

Die einzelnen Strukturelemente sind:

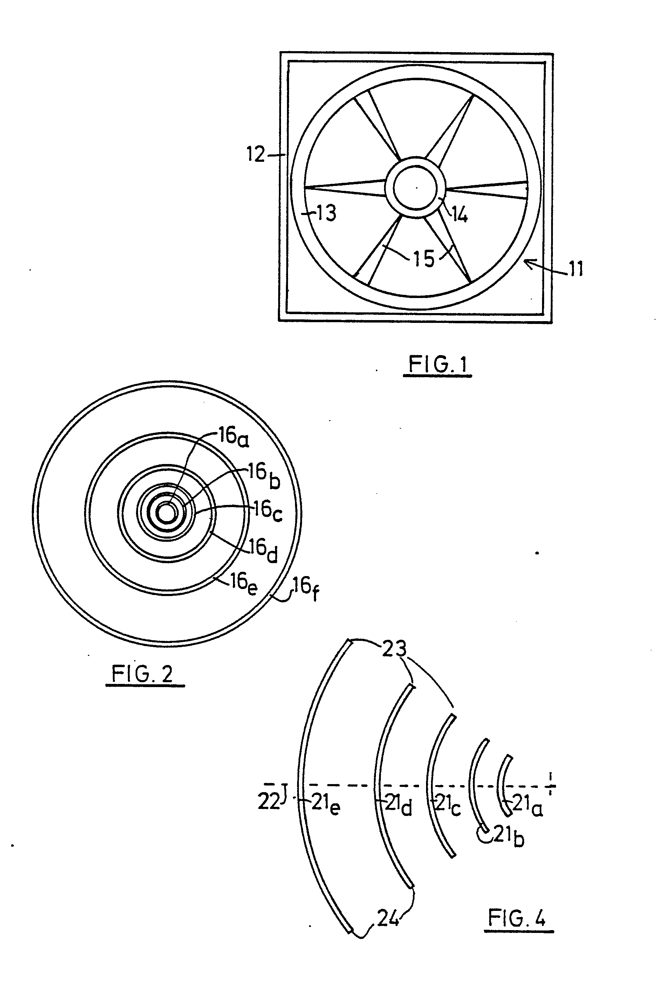

1. Eine dreidimensionale Form in Metall oder einem anderen Stoff von der harmonischen metrischen kugeligen, insbesondere ringförmigen Grundstruktur des Kosmos, wie es klar in dem Meditationsbild von Nikolaus von der Flüe gezeichnet ist (Siehe Figur 1). Die auch teilweise dreidimensionale Ringform ist analog der kugelförmigen und darin ringartigen Grundstruktur des Feldes des Kosmos und aller realen Einheiten in ihm. Entsprechend wirkt sie kausalgesetzlich.

2. In der harmonischen Anordnung des Sonnensystemes zumindest drei, optimal sieben offene oder geschlossene Ringe, die vorzugsweise im mittleren Abstand der sieben Grundplaneten des Sonnensystemes 1/4/7/10/16/52/100 zueinander in einer Ebene angeordnet sind. Diese Figur wird induziert von dem urharmonischen Grundfeld des Sonnensystemes und erzeugt daher transformierend dieses Feld in dem Schutzgerät und um es herum. Jeder Ring kann nach dem Beispiel einer Trafowicklung an seinem Ort auch mehrfach ausgeführt werden, vorzugsweise dreifach.

3. Sieben Grundmetalle, die nach Tradition und Erfahrungswissenschaft den sieben Haupthimmelskörpern des Sonnensystemes zugeordnet sind und die in dieser Ordnung auf die vorbenannten sieben Ringe verteilt sind. Das sind in der Ordnung von innen nach aussen Gold (Sonne), Quecksilber (Merkur), Kupfer (Venus), Silber (Mond als Vertreter der Erde), Eisen (Mars), Zinn (Jupiter) und Blei (Saturn). Diese Metalle werden erfahrungsgemäss von den jeweiligen Planetenfeldern induziert, was sie entsprechend wirken lässt, insbesondere stärker, wenn sie in der planetarischen Reihenfolge angeordnet werden.

Man kann jeweils verschieden legierte Metalle verwenden, vorzugsweise Reinmetalle.

Die beschriebenen Strukturen können in vielerlei Form im Ganzen, verkürzt und auch nur in Teilen angewandt werden.

Ein grosses und hoch wirksames Gerät besteht aus einer Goldkugel in der Mitte und sechs offenen Ringen in den Planetenmetallen, deren Öffnungen sechsstrahlig versetzt sind, auf dessen Gehäuse das sechstrahlige Bild in reinem Gold aufgedruckt ist, wobei die Abstände der Ringe den Abständen der Planetenbahnen entsprechen. Das Quecksilber wird dann vorzugsweise in eine ringsförmige Glasröhre eingeschmolzen.

Literatur zum Nachweis der Wirkungen:
Kepler, Johannes, Weltharmonik, 1982. Wissenschaftliche Buchgesellschaft. Darmstadt 1967.
Palm. Das gesunde Haus. ORDO-Verlag Konstanz. 9. Aufl. 1985
Ostrander. PSI. Scherz-Verlag. In vielen Sprachen.
Toth, Max, Pyramidenkraft (Pyramid power). Goldammer Taschenbuch. .
L. Pfeiffer. Mitteilungen des Biolog. Institutes am Goetheanum Nr. 1 -4.
Stuttgart 1934/35.
Lakhovsky, Georges. Das Geheimnis des Lebens. Kosmische Wellen und vitale Schwingungen. 1981 VGW. Wiss. Nachweis erheblicher mediz. Wirkungen schon durch einen einzigen (!) Schwingungskreis aus Kupfer.

Anwendungen

Dieses Gerät kann zur Erhaltung und Verbesserung der Gesundheit zeitweise oder ständig im Lebensfeld des Menschen verwandt werden. Es kann etwa in Miniform am Körper getragen werden, dann besonders wirksam im Herzbereich mitten auf der Brust oder über dem Solarplexus. Es kann als eigenes Heilgerät verwandt werden und in anderen Heilgeräten zur erheblichen Qualifizierung von deren Wirkung. Es kann auch zusätzlich zur Qualifizierung anderer Heilverfahren und Heilmittel gebraucht werden wie durch nahe Aufstellung bei Heilverfahren am Bett oder Behandlungsort und Aufstellung unter oder über gelagerten Heilmitteln, Nahrungsmitteln und anderen qualifizierten Substanzen.

Das Gerät kann als Computerprotektor verwandt werden bei den zahlreichen noch weitesthin unerklärlichen und sehr verlustreichen Computerstörungen, indem es in harmonischer Anordnung angebracht wird. Es verbessert und stabilisiert die Funktionen des Computers und macht diesen in einem grösseren Temperatur-und Feuchtebereich arbeitsfähig.

Es kann als Schutz-und Entwicklungsgerät für Herstellungsprozesse wie Reifeprozesse, für Nahrungsmittel, Konserven, Getränke, Textilien, Bauten, Bilder, Denkmäler, Antiquitäten, Edelsteine, Metalle, Genussmittel, Kosmetika, Parfüme, Hygienika verwandt werden.

Es kann in teure Geräte wie Fluggeräte, Maschinen, chemische Geräte, Küchengeräte, Ölbrenner, Motoren etc. eingebaut werden. Es kann bei allen Fabrikationsprozessen physikalischer und chemischer Art zur Verbesserung, Schützung, Stärkung und Sicherung der Produktionsgeräte, der Produktion und der Produkte verwandt werden.

Es kann zur Auflösung von Nebelfeldern und zur Harmonierung unerwünschter geologischer Prozesse wie unter Strassen, Bauten, Landefeldern etc. und anderer unerwünschter elementarischer Veränderungen verwandt werden.

Es kann auf ein Fernsehgerät oder Bildschirmgerät gelegt werden oder schon bei der Fabrikation in ihm angebracht werden, umdie gesundheitsschädlichen Wirkungen dessen, was wir bis jetzt technisch als Kathodenstrahlen etc. erfassen, erheblich zu mindern.

Beispielsweise kann es unter ein Gerät zur Bereitung von Speisen, Getränken wie Kaffee, Heil- und Genuss-Tee, Wein, Bier gestellt werden und führt dann zur erheblichen gesundheitlichen wie auch geschmacklichen Qualifizierung der Produkte.

Es kann als universales Harmonierungs-und Entstörgerät bei geopathischen, elektrischen, chemischen und anderen Störzonen verwandt werden.

Es kann bei allen Störungen in den vier Elementen, den drei Ursubstanzen, den zwei Urpolaritäten und in jeder realen Einheit hilfreich verwandt werden.

Weitere Merkmale und Einzelheiten ergeben sich aus der Figur 1 und 2.

Figur 1 zeigt das Meditationsbild von Nikolaus von der Flüe. Im Quadrat 12 liegt ein doppeltes Ringsystem 11 mit dem doppelten Aussenring 13 und Innenring 14. Sie sind mit Hilfe von sechs Speichen 15 miteinander verbunden. Quadrat, Ringsystem und Speichen sind aus einem elektrisch leitenden Metall, vorzugsweise aus Gold.

Figur 2 zeigt eine Ausführungsform, die aus sechs konzentrisch zueinander in einer Ebene angeordneten Ringen 16 besteht. Ring 16a enthält vorzugsweise Gold oder reines Gold. Ring 16 b enthält Quecksiber wie in einem Glasrohr eingeschmolzen. Ring 16 c enthält Kupfer oder nur Reinkupfer. Ring 16 d enthält Silber oder nur Reinsilber. Ring 16 e enthält Eisen oder nur Reineisen.

Ring 16 f enthält Zinn oder nur Reinzinn. Ein nicht dargestellter äusserster Ring enthält Blei oder nur Reinblei.

Die Abstände der Ringe können gleich sein. Vorzugsweise entsprechen sie den Abständen der Planetenbahnen. Normiert man den mittleren Abstand der Erde von der Sonne auf zehn, so betragen die Radien für den Merkur (Quecksilber) 4, für die Venus (Kupfer) 7, für die Erde (Mond/Silber) 10, für den Mars (Eisen) 16, für den Jupiter (Zinn) 52, für den Saturn (Blei) 100 (Reihe von Bode-Titius). Es ist erfindungsgemäss möglich, einen Ring oder mehrere wegzulassen. Jedoch darf die Reihenfolge der Materialien nicht geändert werden.

Bei der Anordnung nach Figur 4 liegen die offenen Ringe als Teilkreise in einer Ebene konzentrisch zueinander, sodass die Metalle oder Metallegierungen der Teilkreise 21 a, b, c, d usf. der Reihenfolge wie bei 16 a, b, c usf. entsprechen. Auch hier können ein Teilkreis weggelassen werden oder mehrere; aber die Reihenfolge der verbleibenden Teilkreise und also ihrer Materialien darf erfindungsgemäss ebenfalls nicht verändert werden gemäss Anspruch 1.

## Ansprüche

1. Schutzgerät, dadurch gekennzeichnet, dass es mindestens drei, vorzugsweise sieben, mindestens teilweise ausgebildete und konzentrisch angeordnete Ringe aufweist, die mindestens in Bereich ihrer Oberfläche elektrisch leitend ausgebildet sind und von denen jeder Ring durch ein anderes Metall der sieben Grundmetalle Gold, Quecksilber, Kupfer, Silber, Eisen, Zinn und Blei elektrisch leitend ist und dass die vorhandenen Ringe in dieser Reihenfolge angeordnet sind.

2. Gerät nach Anspruch 1, dadurch gekennzeichnet, dass von einem inneren Ring sechs Speichen zu einem Doppelring ausgehen, der selber in einem quadratischen Rahmen liegt.

3. Gerät nach einem der vorangehenden Ansprüche, dadurch gekennzeichnet, dass der innerste Ring kugelförmig ausgebildet ist.

4. Gerät nach einem der vorangehenden Ansprüche, dadurch gekennzeichnet, dass die Abstände der konzentrisch liegenden Ringe den Abständen der Planetenbahnen des Sonnensystemes entsprechen.

5. Gerät nach einem der vorangehenden Ansprüche, dadurch gekennzeichnet, dass Öffnungen der Ringe versetzt angeordnet sind.

FIG.1

FIG. 2

FIG. 4

| | **EINSCHLÄGIGE DOKUMENTE** | | EP 86111979.0 |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| A | FR - A1 - 2 554 354 (DUPIN) <br> * Zusammenfassung; Seite 3, Zeilen 6-23; Fig. 2 * <br> -- | 1 | A 61 N 1/16 |
| A | DE - A1 - 3 320 510 (VÖLKNER) <br> * Seite 1, Anspruch 1; Seite 5, Positionen 28-30 * <br> -- | 1,5 | |
| A | GB - A - 1 077 765 (ANFOSSO) <br> * Fig. 1-3 * <br> -- | 1 | |
| A | GB - A - 766 886 (EDWARDS) <br> * Seite 2, Ansprüche 1,2; Fig. * <br> -- | 1,5 | |
| A | DE - A1 - 3 320 518 (SCHWEITZER) <br> * Fig. 1-4 * <br> -- | 1 | RECHERCHIERTE SACHGEBIETE (Int. Cl.4) <br><br> A 61 N |
| A | EP - A2 - 0 098 656 (GIERKINK) <br> * Zusammenfassung; Fig. 1-9 * <br> ---- | 1 | |

Der vorliegende Recherchenbericht wurde für alle Patentansprüche erstellt.

| Recherchenort | Abschlußdatum der Recherche | Prüfer |
|---|---|---|
| WIEN | 31-03-1987 | NEGWER |